# EUROPEAN PATENT APPLICATION

(11) **EP 2 295 998 A1**
(43) Date of publication of application: **16.03.2011**
(21) Application number: 09169950.4
(22) Date of filing: 10.09.2009
(51) Int. Cl.: G01R 33/28, A61K 49/06, A61B 5/055, G01R 33/62, G01R 33/3815

(54) **Magnetic resonance imaging system and magnet comprising a polarizer**

(71) Applicant: Koninklijke Philips Electronics N.V., 5621 BA Eindhoven (NL); Philips Intellectual Property & Standards, 20099 Hamburg (DE)
(72) Inventor: The designation of the inventor has not yet been filed
(74) Representative: Damen, Daniel Martijn

(57) **Abstract**

A magnetic resonance imaging system (100) adapted for acquiring magnetic resonance images comprising:
- a magnet (102) adapted for generating a magnetic field (262) for orientating the magnetic spins of nuclei of a subject (104) located within an imaging region (108),
- a polarizer (120) adapted for polarizing a hyperpolarizable contrast agent (484), wherein the polarizer is located within a polarization field region (118) of the magnetic field of the magnet,
- a radio frequency system (110) comprising a coil (114) adapted for acquiring magnetic resonance imaging data from the hyperpolarizable contrast agent,
- a magnetic field gradient coil (114) adapted for spatial encoding the magnetic spins of nuclei,
- a magnetic field gradient coil power supply (116) adapted for supplying current to the magnetic field gradient coil,
- a computer system (130) adapted for constructing images from the magnetic resonance imaging data and for controlling the operation of the magnetic resonance imaging system.

## Description

### TECHNICAL FIELD

The invention relates to equipment used for acquiring magnetic resonance imaging images of hyperpolarized contrast agents, in particular the integration of the polarizer into a magnetic resonance imaging system or a magnet for magnetic resonance imaging.

### BACKGROUND OF THE INVENTION

A static magnetic field is used by Magnetic Resonance Imaging (MRI) scanners to align the nuclear spins of atoms as part of the procedure for producing images within the body of a patient or subject. This large static magnetic field is referred to as the B₀ field.

Magnetic resonance imaging systems or scanners typically are used to image the concentrations or properties of protons, or hydrogen atoms, in a subject. Magnetic resonance imaging systems are especially useful for imaging the soft tissues of a subject. Contrast agents are often used to enhance imaging.

The journal article Goldman et al., "Molecular imaging using hyperpolarized 13C", The British Journal of Radiology, 76 (2003), S118-127 reviews the use of ¹³C as a hyperpolarized contrast agent for medical imaging.

A disadvantage with the current technology is that many hyperpolarized contrast agents have a very short relaxation time such that a substantial portion of the contrast agent will lose its polarization before being administered to a subject. For example, hyperpolarized contrast agents which contain ¹³C such as pyruvate may have a T₁ relaxation time of the order of 60 seconds. Due to this fast decay of the degree of polarization, it would be advantageous to decrease the amount of time required to administer the hyperpolarized contrast agent to the subject as soon as the agent has reached its final state of polarization.

### SUMMARY OF THE INVENTION

The invention provides for a magnetic resonance imaging system and a magnet in the independent claims. Embodiments of the invention are given in the dependent claims.

Embodiments of the invention may have the advantage that the magnetic field produced by the magnetic resonance imaging system is used by the polarizer; this eliminates the need for a separate magnet for the polarizer.

Further embodiments of the invention may have the advantage of reducing the amount of time to administer the hyperpolarized contrast agent to a subject by integrating the polarizer for the hyperpolarized contrast agent into the magnetic resonance imaging system. A polarizer for polarizing a hyperpolarized contrast agent may require that expensive and bulky equipment needs to be placed in the vicinity of a magnetic resonance imaging scanner. Embodiments of the invention may reduce the amount of equipment needed to implement a polarizer by using or reusing equipment already present at the site of a magnetic resonance imaging system. For example various embodiments may use the magnetic field generated by the magnet used to generate the B₀ field, the cryogenic cooling of the magnet used to generate the B₀ field, and parts of the radio frequency (RF) system that comprise a RF-spectrometer used for imaging may be used to monitor the polarization level of a hyperpolarizable contrast agent during polarization.

When a polarized hyperpolarizable contrast agent passes through a magnetic field gradient some polarization may be lost. A particularly advantageous benefit that embodiments of the invention may have is that when the polarizer is integrated into the magnetic resonance imaging system and/or magnet, the magnetic field B₀ gradient that the hyperpolarizable contrast agent experiences can be minimized. Bringing a polarized hyperpolarizable contrast agent into a low or zero magnetic field region and then bringing it into a high magnetic field region will also reduce or destroy its polarization. Simply being located in a polarization field region of the magnetic resonance imaging system's magnet may yield these advantages because the hyperpolarizable contrast agent is polarized in the same magnetic field in which it will be administered to a subject. Additionally, embodiments may amplify this advantage by altering the design of coils used to generate the magnetic field or by placing one or more ferromagnetic structures between the polarizer and the region in which the sample will be used.

Some embodiments may have the advantage of being able to integrate the cooling system of the polarizer with the cooling system of the magnet.

As used herein, the term hyperpolarizable contrast agent refers to a hyperpolarizable material that is used as a contrast agent during the acquisition of magnetic resonance imaging data. The hyperpolarized contrast agent may be in gaseous, liquid or solid form before it is administered. Before use, the hyperpolarized contrast agent may be polarized using a process such as Dynamic Nuclear Polarization (DNP) or any other suitable process. The polarized nuclei of the hyperpolarized contrast agent may be embedded into a biocompatible molecule. Isotopes that may be used to make hyperpolarized contrast agents include: ⁷Li, ¹³c ¹⁴N, ¹⁵N, ¹⁷O, ¹⁹F, ²³Na, ³¹P, ⁸¹Y. Since it is beneficial to embed the isotope into a biocompatible molecule ¹³C is of particular interest, due to the presence of carbon in many biological molecules. ¹⁵N and ¹⁷O also offer the possibility of being more easily integrated into biocompatible molecules.

As used herein, the term magnetic field B₀ gradient is the calculated gradient of the B₀ magnetic field produced by the magnet.

As used herein, the term coil refers to a radio frequency antenna that is used to acquire magnetic resonance imaging data. The term coil may refer to the case where a single antenna performs both the transmission and reception of radio frequency signals during the acquisition of magnetic resonance imaging data or it may refer to a transmit and an receive antenna. Moreover an array configuration for either case mentioned consisting of a multitude of single elements is considered.

Magnetic Resonance Imaging (MRI) data is defined herein as being the recorded measurements of radio frequency signals emitted by atomic spins by the antenna of a magnetic resonance apparatus during a magnetic resonance imaging scan. A Magnetic Resonance Imaging(MRI) image is defined herein as being the reconstructed two or three dimensional visualization of anatomic data contained within the magnetic resonance imaging data. This visualization can be performed using a computer system.

Nuclear Magnetic Resonance (NMR) is synonymous with Magnetic Resonance. The term Nuclear Magnetic resonance is used herein with reference to using the radio frequency system used to acquire magnetic resonance imaging data to determine the degree of polarization that a hyperpolarizable contrast agent in a polarizer has.

A computer system is defined herein as a computer or a collection of computers. For magnetic resonance imaging, a single computer may be used to operate and perform analysis of the magnetic resonance imaging data. However, this functionality is often distributed across many different computers, and the magnetic resonance imaging data may be stored for analysis by a computer or computer system later.

A polarizer is defined herein as an apparatus for polarizing a hyperpolarizable contrast agent. A polarizer may function in different ways. A particularly common way is the use of Dynamic Nuclear Polarization (DNP). At low temperatures, about 1 Kelvin, and at high magnetic fields, about 3 Tesla, a hyperpolarized contrast agent is doped with a single-electron substance. Microwave irradiation near the electron resonance frequency leads first to a polarization of the single electron substance and then to a transfer of the polarization from the single electron substance to the nuclei of the hyperpolarized contrast agent. At such low temperatures, the hyperpolarizable contrast agent and the single electron substance may be are solid. In this case a heating system or warm water system may be used to melt the hyperpolarized contrast agent before use. Alternatively the hyperpolarized contrast agent may be removed from the polarizer in solid form.

A hyperpolarizable contrast agent dispenser, as used herein, is a apparatus for dispensing hyperpolarizable contrast agent to a subject. The hyperpolarizable contrast agent may be dispensed in liquid form, gaseous form, or in a vapor. When dispensed in liquid form the hyperpolarizable contrast agent dispenser may comprise a connection adapted for injecting the hyperpolarized contrast agent into the subject. The hyperpolarized contrast agent may also comprise a filtration system for filtering the hyperpolarized contrast agent before dispensing it to the subject. The filtration system may be adapted for removing toxic or poisonous chemicals from the hyperpolarized contrast agent before dispensing the hyperpolarized contrast agent to the subject.

A 1 Kelvin pot or 1-K pot is a cryogenic device which is used to achieve low temperatures using liquid helium. A polarizer may comprise a 1 Kelvin pot in order to bring the hyperpolarized contrast agent to low enough temperatures to perform the polarization.

In the imaging of hyperpolarized compounds, time is a critical factor, since as was mentioned before the polarization typically decays rapidly once the compound is heated up. Therefore, the transfer from the polarizer to the patient and the administration has to be performed quickly. Embodiments of the invention may have the following advantages:
- An integrated polarizer can reduce the time from preparation to administration, as the sample is close to the MRI system.
- An integrated polarizer can serve as an ideal storage system for polarized compounds (stored at He temperature and ready for use).
- No separate magnet and infrastructure leads to a compact system for cost-effective and efficient clinical solutions.
- Diagnostic MR measurements and NMR and EPR data acquisitions to monitor the quality of the sample to be hyperpolarized can be done with same MR spectrometer.
- One software application controls the whole process of sample production and administration.

The invention provides for a magnetic resonance imaging system adapted for acquiring magnetic resonance imaging images. The magnetic resonance imaging system comprises a magnet for generating a magnetic field for orientating magnetic spins of nuclei of a subject located within an imaging region. The magnet may be a superconducting, a resistive magnet or a combination of superconducting and resistive magnets. A toroidal magnet or a so-called open magnet may also be used.

The imaging region, as used herein, is a region with a magnetic field that is uniform enough that magnetic resonance imaging can be performed. A subject or object to be imaged using a magnetic resonance imaging system is placed or partially placed within the imaging region. The magnetic resonance imaging system further comprises a polarizer adapted for polarizing a hyperpolarizable contrast agent. The polarizer is located within a polarization field region of the magnetic field of the magnet. The polarization field region, as used herein, is a region with a magnetic field of sufficient strength and homogeneity for performing polarization of a hyperpolarized contrast agent using a polarizer.

The magnetic resonance imaging system further comprises a radio frequency system comprising a coil calibrated for acquiring magnetic resonance imaging data from the hyperpolarizable contrast agent. The magnetic resonance imaging system further comprises a magnetic field gradient coil for spatial encoding of the magnetic spins of the nuclei.

The magnetic resonance imaging system further comprises a magnetic field gradient coil power supply for supplying current to the magnetic field gradient coil. The magnetic resonance imaging system further comprises a computer system for constructing images from the magnetic resonance imaging data and for controlling the operation of the magnetic resonance imaging system. By controlling the operation, it is understood that the computer system is adapted for controlling at least the radio frequency system and the magnetic field gradient coil power supply. In some embodiments the polarizer is controlled by the computer system also.

This magnetic resonance imaging system is advantageous, because the polarizer is located within the polarization field region of the magnetic field of the magnet. If material is hyperpolarized in a high magnetic field and then removed from this field some of the polarization may be lost due to large magnetic field gradients. In the case where the polarizer is located out of the polarization field region of the magnet, the material is polarized, brought out of the high magnetic field and then brought into the high magnetic field of the magnetic resonance imaging system. This often causes a loss of polarization. The hyperpolarized contrast agent is polarized within the polarization field region, and does not experience such large magnetic field gradients. In addition the hyperpolarizable contrast agent is located closer to a subject or object which may be within the imaging region. In this case the hyperpolarizable contrast agent can be administered more rapidly than if the contrast agent were further away.

In another embodiment, the polarizer is adapted for being connected to a hyperpolarizable contrast agent dispenser. This embodiment is advantageous, because a polarizer can be connected directly to a hyperpolarizable contrast agent dispenser. This is advantageous, because it allows the hyperpolarizable contrast agent to be produced with an improved workflow. The hyperpolarizable contrast agent dispenser can be connected to a subject and configured to automatically inject the subject with the hyperpolarizable contrast agent after it has been polarized in the polarizer.

In another embodiment, the computer system is further adapted for sending control signals to the dispenser. The computer system is further adapted for controlling the polarization of the hyperpolarizable contrast agent. The computer system is further adapted for controlling the dispensing of hyperpolarizable contrast agent to a subject using the polarizer and the dispenser. This embodiment is particularly advantageous, because the polarization of the hyperpolarizable contrast agent and its delivery to a subject is completely automated. This allows a much faster workflow. Hyperpolarizable contrast agents have their polarization decay very rapidly so this would increase the quality of the images produced.

In another embodiment, the polarizer comprises a nuclear magnetic resonance coil adapted for receiving nuclear magnetic resonance data from the hyperpolarizable contrast agent. The radio frequency system is adapted for acquiring nuclear magnetic resonance data from the hyperpolarizable contrast agent using the nuclear magnetic resonance coil. This embodiment is advantageous, because the need for a separate radio frequency system for the acquisition of the nuclear magnetic resonance data is not needed.

This embodiment may be implemented in several different ways. One example is the use of a radio frequency system with multiple channels. One channel may be dedicated to measuring the polarization and the remaining channels may be used for acquiring the magnetic resonance imaging data. Another example is the multiplexing of a channel of the radio frequency system. A channel may use a multiplexer such that at one time the channel of the radio frequency system is used to measure the polarization of hyperpolarizable contrast agent within the polarizer and during a different time period the channel of the radiofrequency system is used to acquire magnetic resonance imaging data. These two examples are not mutually exclusive: For example, a multi channel radio frequency system may be constructed where one of the channels is multiplexed.

In another embodiment, the radio frequency system has at least two channels for acquiring magnetic resonance imaging data. The polarizer comprises a nuclear magnetic resonance coil adapted for receiving nuclear magnetic resonance data from the hyperpolarizable contrast agent. One of the at least few channels of the radio frequency system is adapted for acquiring nuclear magnetic resonance data from the hyperpolarizable contrast agent using the nuclear magnetic resonance coil. This embodiment is advantageous, because the need for a separate radio frequency system for the acquisition of the nuclear magnetic resonance data is not needed.

In another embodiment, the magnetic resonance imaging system further comprises an electron paramagnetic resonance spectrometer for monitoring the polarization of the hyperpolarizable contrast agent. This embodiment is advantageous, because this allows the monitoring of the degree of polarization of the hyperpolarizable contrast agent. The monitoring of the degree of polarization of the hyperpolarizable contrast agent may be achieved by monitoring the degree of polarization of the single electron substance.

In another embodiment, the computer system is further adapted for controlling the polarizer wherein the computer system comprises a display. The computer system is further adapted for displaying the polarization level of the hyperpolarizable contrast agent in the polarizer. This embodiment is advantageous, because it allows an operator or a user to monitor the polarization level of the hyperpolarizable contrast agent.

In another aspect, the invention provides for a magnet for generating a magnetic field for orienting the magnetic spins of nuclei of a subject located within an imaging region for a magnetic resonance imaging system. The magnet further comprises at least one polarizer adapted for polarizing a hyperpolarizable contrast agent. The polarizer is located within a polarization field region of the magnetic field of the magnet. This embodiment is advantageous, because a large magnetic field is needed for polarizing a hyperpolarizable contrast agent. Locating the polarizer as a component or part of the magnet allows the polarizer to be placed within a suitably large magnetic field generated by the magnet. This allows the elimination of a separate magnet for the polarizer. In addition, when a polarized hyperpolarizable contrast agent experiences a magnetic field gradient some polarization is lost. The polarizer can be located within the polarization field such that the magnetic field gradient that the polarized hyperpolarizable contrast agent experiences is minimized. In this way the polarization of a hyperpolarizable contrast agent can be maximized when it is administered to a subject.

In another embodiment, the polarizer is one of: a dynamic nuclear polarization polarizer, an optical polarizer, a microwave polarizer, a hybrid optical and microwave polarizer, a PHIP (Para-Hydrogen Induced Polarization) polarizer, and a polarizer adapted for hyperpolarizing multiple types of nuclei.

In another embodiment, there is more than one polarizer.

In another embodiment, the magnet comprises a patient support, wherein the polarizer is located below the patient support. The polarizer may be contained within the patient support, it may be located below the patient gantry, or it may be integrated into the body of the magnet below the patient support.

In another embodiment, the magnet comprises a drawer adapted for pulling the polarizer and/or adapted for storing hyperpolarizable contrast agent. This embodiment is advantageous, because a drawer can be constructed in a location with a high magnetic field and the drawer allows for the convenient placing of the hyperpolarizable contrast agent into the polarizer or into a system for loading the hyperpolarizable contrast agent into the polarizer. For instance a cartridge system containing hyperpolarized contrast agent could be used in the drawer.

In another embodiment, the polarizer comprises a 1 Kelvin pot. The magnet comprises a cryogenic cooling system for cooling the magnet and the 1 Kelvin pot.

In another embodiment, the polarizer comprises a 1 Kelvin pot. The magnet has a cryogenic cooling system and the 1 Kelvin pot is integrated into the cooling system of the magnet. The magnet further comprises a cryogenic cooling system. This embodiment is advantageous, because the polarizer does not need its own cooling system.

In another embodiment, the magnet comprises more than one polarizer.

In another embodiment, the magnet further comprises a ferromagnetic structure that follows a path at least partially between the imaging region and the polarization field region. The ferromagnetic structure is adapted for minimizing the magnetic field B₀ gradient between the polarization field region and the imaging region and/or the ferromagnetic structure is adapted for enhancing a local region of the magnetic field between the polarization field region and the imaging region This embodiment is particularly advantageous because the ferromagnetic structure affects the magnetic field and the hyperpolarizable contrast agent can be transported along the path between the polarization field region and the imaging region. By following the path the magnetic field B₀ gradient between the imaging region and the polarization field region may be minimized. This allows the polarization of the hyperpolarizable contrast agent when it reaches the imaging region to be as large as possible.

In another embodiment, the magnet has a cryostat. The polarization field region is within the cryostat. This embodiment is advantageous, because the polarizer is then located within the cryostat. In this case the polarizer and the magnet share a cooling system.

In another embodiment, the magnet is further adapted such that the magnitude of the magnetic field generated in the polarization field region is stronger than the magnitude of the magnetic field generated in the imaging region. This is advantageous, because the higher magnetic field may allow the polarizer to achieve a higher degree of polarization when polarizing a hyperpolarizable contrast agent.

In another aspect, the invention provides for a patient support for a magnetic resonance imaging system. The patient support comprises a polarizer adapted for polarizing a hyperpolarizable contrast agent. By being located within the patient support the polarizer can be positioned such that the polarizer is located in a polarization field region of the magnet when the patient support is installed into a magnetic resonance imaging system. This arrangement has the same advantages as which were discussed when the polarizer is located as a part of a magnet for generating a magnetic field for a magnetic resonance imaging system. The patient support may have its own cooling system for the polarizer, or it may be connected to the cooling system of the magnet.

In another embodiment, the subject support is adapted for being integrated into a magnet adapted for generating a magnetic field for orientating the magnetic spins of nuclei of a subject located within an imaging region.

### BRIEF DESCRIPTION OF THE DRAWINGS

In the following preferred embodiments of the invention will be described, by way of example only, and with reference to the drawings in which:
Fig.1 shows a function diagram of a magnetic resonance imaging system according to an embodiment of the invention,
Fig. 2a shows calculated magnetic fields adjacent to coils in a superconducting magnetic resonance imaging magnet according to an embodiment of the invention,
Fig. 2b shows the design of a magnet according to an embodiment of the invention that incorporates the coils shown in Fig 2a,
Fig. 2c shows the design of a magnet according a further embodiment of the invention that incorporates the coils shown in Fig 2a,
Fig 2d shows the design of a magnet according a further embodiment of the invention that incorporates the coils shown in Fig 2a,
Fig. 3 illustrates a portion of a magnetic resonance imaging system according to an embodiment of the invention where the polarizer is located within a drawer,
Fig. 4 presents a polarizer located in the bore of a magnet according to an embodiment of the invention,
Fig. 5 illustrates a polarizer integrated into a patient support according to an embodiment of the invention,
Fig. 6 shows a further functional diagram of a magnetic resonance imaging system according to an embodiment of the invention,
Fig. 7 shows a workflow diagram for using a magnetic resonance imaging system according to an embodiment of the invention, and
Fig. 8 illustrates a polarizer integrated into the cryostat of a magnet according to an embodiment of the invention.

### DETAILED DESCRIPTION OF THE EMBODIMENTS

Like numbered elements in these figures are either identical elements, equivalent elements, or perform the same function. Elements which have been discussed previously will not necessarily be discussed in later figures if the function is equivalent.

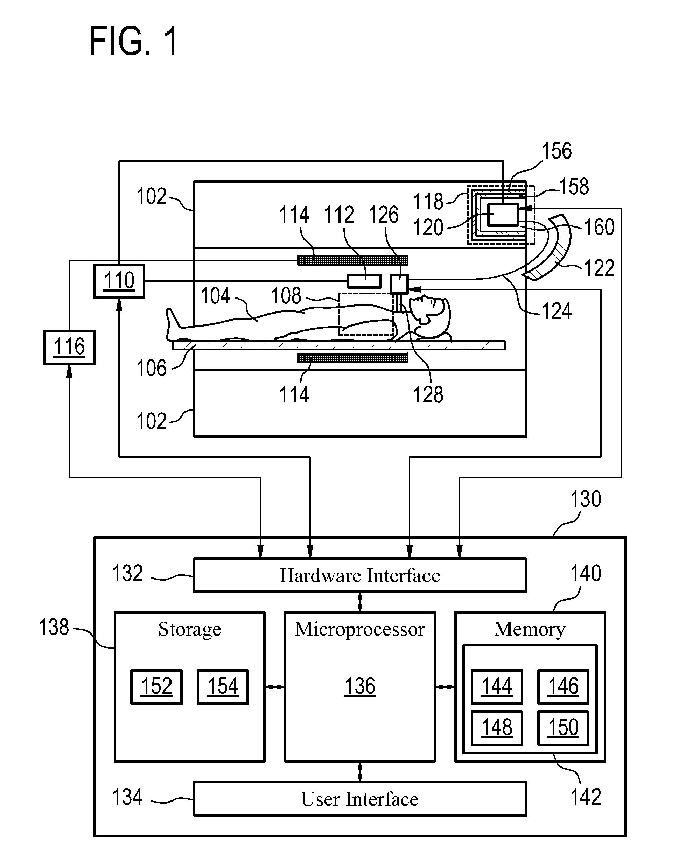

Fig. 1 shows a magnetic resonance imaging system 100 according to an embodiment of the invention. There is a magnet 102. The magnet 102 is shown as a cross-sectional view of a cylindrical magnet. Other magnets such as the so called open magnets which are configured like Helmholtz coils are also applicable. Within the bore of the magnet 102 is a subject 104 located on a subject support 106. A portion of the subject 104 is located within the imaging region 108. Within the imaging region 108 the magnetic resonance imaging system 100 is adapted for acquiring magnetic resonance imaging data.

There is a radio frequency system 110 attached to a coil 112. The coil is adapted for exciting the magnetic spins within the imaging region 108. There is a set of gradient coils 114 which is attached to a magnetic field gradient power supply 116. The magnetic field gradient coil 114 is adapted for spatially encoding magnetic spins within the imaging region 108.

The dashed box 118 shows a polarization field region 118. Within the polarization field region 118 is a magnetic field which is strong enough for a polarizer 120 to operate. The polarizer 120 is adapted for polarizing a hyperpolarizable contrast agent. There is a tube 124 between the polarizer 120 and a hyperpolarizable contrast agent dispenser 126. The tube is adapted for transporting liquid hyperpolarizable contrast agent between the polarizer 120 and the hyperpolarizable contrast agent dispenser 126. In solid form, hyperpolarizable contrast agent could also be moved manually or by a mechanical system between the polarizer 120 and the hyperpolarizable contrast agent dispenser 126.

When transporting the hyperpolarizable contrast agent from the polarizer 120 to the hyperpolarizable contrast agent dispenser 126 several factors need to be considered:
1. The direction of the polarization should not be changed rapidly. Sharp bends of the tube 124 carrying the hyperpolarizable contrast agent or magnetic field line reversals along the tube are to be avoided or the velocity of the hyperpolarizable contrast agent in the tube 124 should remain small enough so that the spins can keep aligned with the field at all times.
2. The hyperpolarizable contrast agent should not pass through regions of zero magnetic field.

Adjacent to the tube 124 is a ferromagnetic structure 122. A purpose of the ferromagnetic structure 122 is to reduce the magnetic field gradient between the dispenser 120 and the hyperpolarizable contrast agent dispenser 126. The hyperpolarizable contrast agent dispenser 126 is connected to an intravenous connection between the hyperpolarizable contrast agent dispenser 126 and the subject 104. In this way this embodiment can be run fully automatically where the polarizer 120 is controlled by a computer system 130 and the dispenser 126 is also controlled by the computer system 130. The dispenser 126 puts the hyperpolarizable contrast agent into a form which is suitable for injection or administration to the subject 104. In some cases the hyperpolarizable contrast agent contains toxic materials which need to be filtered before they are administered to the subject 104.

In the embodiment shown in Fig. 1, it can be seen that the polarizer is accessible from outside the magnet 102. The polarizer is located within a cavity 160 of the magnet. The cavity 160 has an inner wall 156 and an outer wall 158. The inner wall 156 is an extension of the outer vacuum shell of the cryostat. The outer wall 158 may comprise a thermal radiation shield. The outer wall 158 is an extension of the helium tank of the magnet 102. The inner wall 156, the radiation shield, and the outer wall 158 do not touch, and the space between them form part of the magnet's insulating vacuum. Construction details are given in later figures.

The tube 124 follows a path such that it follows as closely as possible the field lines of the magnet. The ferromagnetic structure 122 is used in this embodiment to alter the location of local magnetic field lines.

A ferromagnetic structure 122 is also useful in the situation where the path of the tube goes through a region where the magnetic field is too low. For example the magnetic field drops below 0.3 Tesla. In this case a ferromagnetic structure may be used to enhance the local magnetic field.

Item 130 is a computer system. The computer system may be a single computer or it may be a network of computers that work together cooperatively. The computer system has a hardware interface 132 which is adapted for controlling the radio frequency system 110, the magnetic field gradient power supply 116, the polarizer 120 and the dispenser 126. Also shown in this figure is a connection between the radio frequency system 110 and the polarizer 120. The connection between the radio frequency system 110 and the polarizer 120 may be a separate channel of the radio frequency system or it may represent a multiplexed channel. The radio frequency system 110 is used to acquire data on a nuclear magnetic resonance coil for the purpose of determining the degree of polarization of any hyperpolarizable contrast agent being polarized in the polarizer 120.

The hardware interface 122 is connected to a microprocessor 136. The microprocessor 136 could be a microcontroller, a microprocessor or a collection of microprocessors or even multiple multi-core microprocessors. The microprocessor is connected to computer storage 138. The storage could be a hard drive, it could be a solid state drive, it could be external storage such as a USB device, an optical disc or a floppy disc. The microprocessor is also connected to computer memory 140. Additionally the microprocessor 136 is also connected to a user interface 134.

The user interface 134 may comprise a display adapted for displaying information or graphics for an operator. The user interface may also contain equipment for inputting data such as a keyboard, a mouse, a touch pad, or a digitizer. Within the memory 140 of the computer is a computer program product 142. The computer program product 142 contains machine executable instructions for operating the magnetic resonance imaging system 100. Module 144 is an image construction module, and is used for taking magnetic resonance imaging data and constructing visualizations of it. Module 146 is a magnetic resonance imaging system control module. The magnetic resonance imaging system control module 146 contains machine executable instructions for controlling the operation of the magnetic resonance imaging system 100. Module 148 is a hyperpolarizable contrast agent dispenser control module. The hyperpolarizable contrast agent dispenser control module 148 contains machine executable instructions for controlling the operation of the dispenser 120. Module 150 is a polarizer control module. The polarizer control module 150 contains machine executable instructions for controlling the operation of the polarizer.

The computer storage 138 may store various types of data. For instance the storage may contain a computer program product 152 for operating the magnetic resonance imaging system 100. The software modules 144, 146, 148, 150 represented in the computer program product 142 may be stored in the storage 138 of the computer system 130. The storage 138 may also contain an archive of patient data 154. The archive of the patient may include but is not limited to magnetic resonance imaging data, images calculated from magnetic resonance imaging data, and log data associated with examinations of various subjects 104.

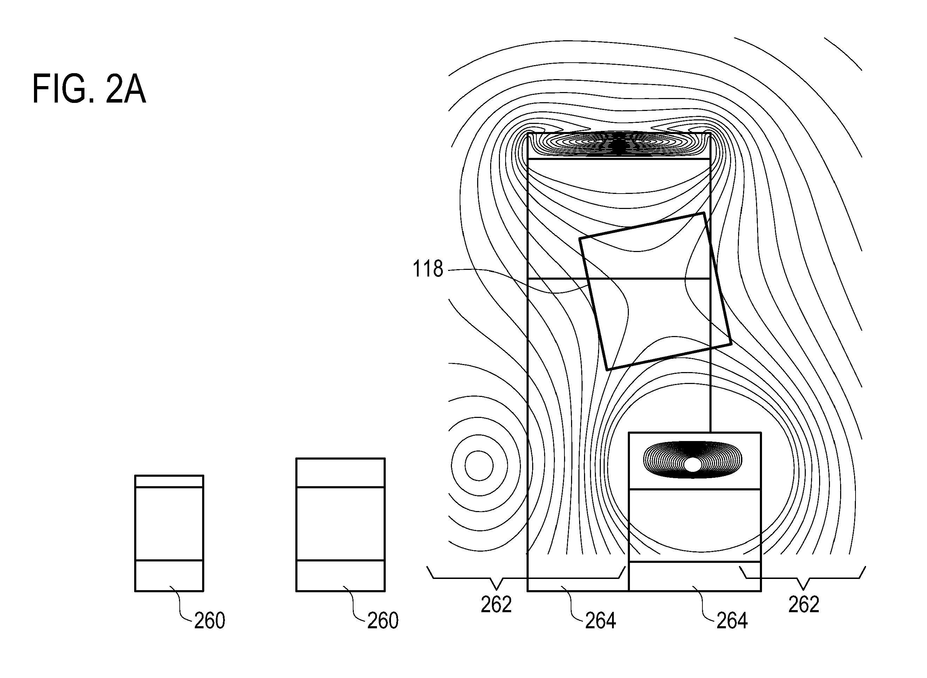

Fig. 2a demonstrates how a polarizer could be placed within a polarization field region 118 of a magnet for a magnetic resonance imaging system. In Fig. 2a is shown a cross-sectional view of superconducting coils 260, 264 which comprise a part of a magnet for a magnetic resonance imaging system. Superimposed on two of the coils 264 are calculated field lines 262. There is a polarization field region 118 between these two coils 264 with the field lines 262 drawn. In the polarization field region 118 the polarization field region is shown in this example as having a field above 2 Tesla or higher. The magnet in this example has only a field of 1.5 Tesla in the imaging region. This figure demonstrates how the magnetic field strength of the polarization field region may be larger than the magnetic field strength in the imaging region. Additional coils can also be added to increase the uniformity of the magnetic field in the polarization field region 118. The magnet shown in Fig 2a is cylindrical. The cross sectional view is only of the top. If a subject were in the magnet, the region shown would be above the subject.

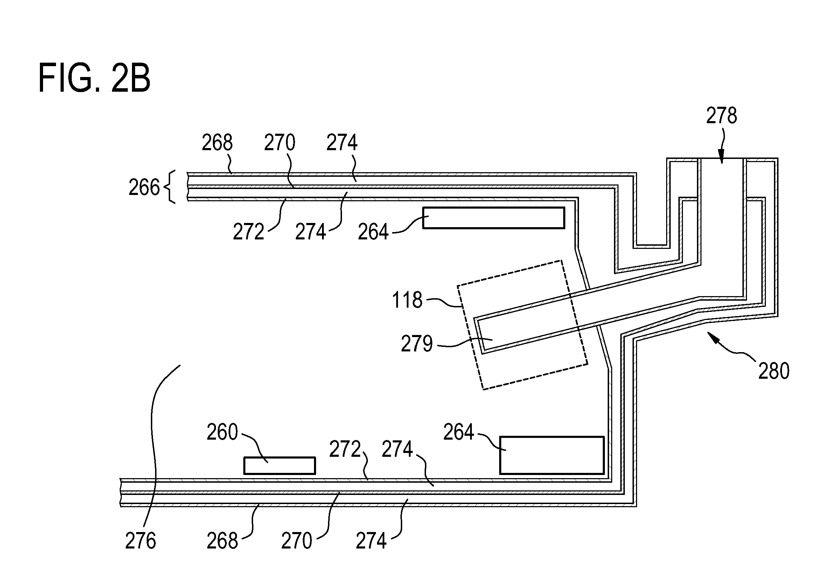

Fig. 2b shows the design of a magnet according to an embodiment of the invention that incorporates the coils shown in Fig 2a. Fig. 2b shows construction details for a magnet according to an embodiment of the invention which uses the coil arrangement shown in Fig. 2a. Coil 260 is shown and the two coils labeled 264 which were used for calculating the field lines shown in Fig. 2a are also shown. For reference, the polarization field region 118 is also shown. The interior of the magnet is labeled 276. The design of the magnet shown in Figs. 2a and 2b is a cylindrical magnet. The view shown in Fig. 2b is a cross-sectional view. Only the top section of the magnet is shown. If a subject were within the bore of the magnet, the region shown in Fig. 2b would be the portion of the magnet above the subject. Surrounding the interior of the magnet 276 is the wall of the magnet 266. The wall of the magnet 266 comprises a cryogenic shield. The wall 266 comprises an exterior wall 268, a radiation shield 270 and an interior wall 272. The radiation shield 270 may be cooled by means of a refrigerator to approximately 50 Kelvin. In between the exterior wall 268 and the radiation shield 270 is a vacuum 274. Also in between the radiation shield 270 and the interior wall 272 is also a vacuum 274. In the design shown in Fig. 2b the 1 Kelvin pot 280 is shown as being integrated into the magnet. An entrance to the 1 Kelvin pot 278 is also shown. In this embodiment the polarizer is located within the 1 Kelvin pot in tube 279. The polarizer is located within the polarization field region 118.

The tube 279 penetrating the polarization field region 118 is filled with liquid helium, which is reduced in temperature to 1K by pumping it. Outside of the magnet the tube 279 has a vertical extension, to prevent the liquid helium from flowing out. Inside the magnet the 1 Kelvin pot 280 is exposed to the liquid helium within the main magnet tank 276 without any significant insulation in between. The 1 Kelvin pot 280 has to penetrate the outer vacuum tank and the radiation shield in such a way that the vertical extension on the outside of the magnet is also thermally insulated, up to a height at least 200 mm above the highest helium level inside the tube.

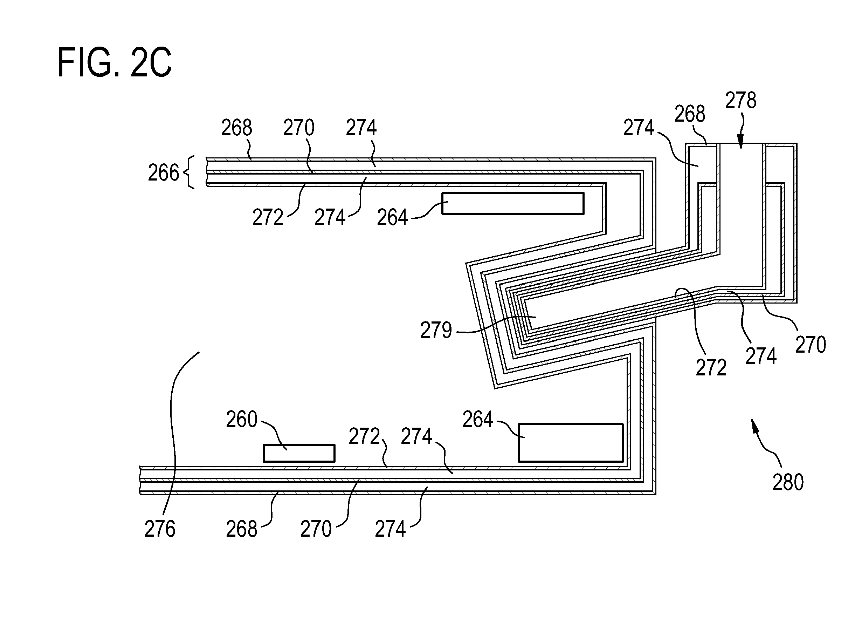

Fig. 2c shows the design of a magnet according a further embodiment of the invention that incorporates the coils shown in Fig 2a. Fig. 2c shows another embodiment of a magnet according to the invention which integrates a polarizer. The design in Fig. 2c is similar to the design shown in Fig. 2b. Instead of the 1 Kelvin pot 280 being integrated into the magnet, the polarizer would be located withing tube 279 within a 1 Kelvin pot 280 that is removable. It can be seen in this figure that the 1 Kelvin pot 280 is thermally isolated from the magnet. In this embodiment, the polarizer can be serviced without bringing the magnet to room temperature.

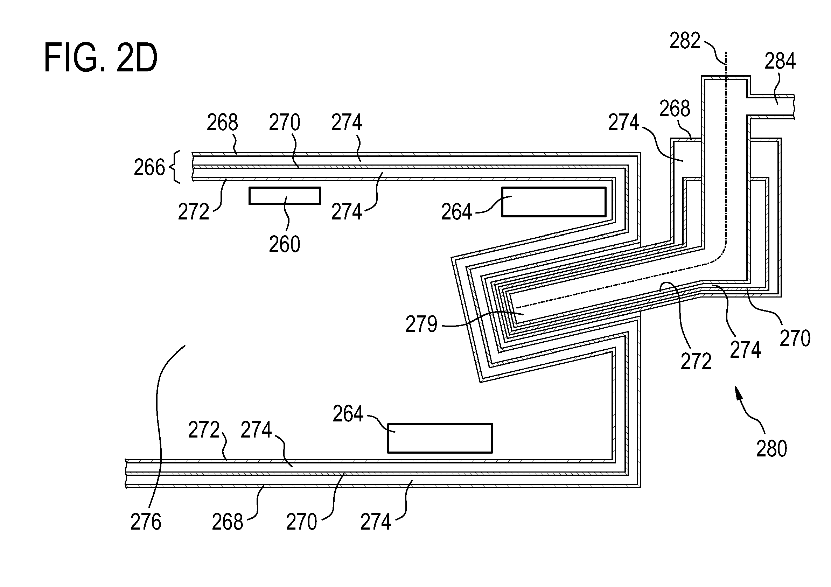

Fig 2d shows the design of a magnet according a further embodiment of the invention that incorporates the coils shown in Fig 2a. The magnet shown in Fig. 2d is similar to that shown in Figs. 2b and 2c except that the region of the magnet is in this case below the subject. It can be noticed that the positioning of the magnets is a mirror image to that shown in Figs. 2a, 2b and 2c. As was shown in the embodiment in Figs. 2c and 2b, the polarizer can be installed via tube 279 into a 1Kelvin pot 280. Being below the subject, the hyperpolarized contrast agent can be removed along a path which better approximates the field lines of the magnet. Dashed line 282 shows a proposed path. This path would not be exactly along the field lines, but it would better approximate the field lines in the embodiments shown in Figs. 2b and 2c. The polarizer in the embodiment in Fig. 2d could also be concealed by a patient support. Outlet 284 is a gas outlet for the 1 Kelvin pot 280.

In both Fig. 2c and Fig. 2d, the 1 Kelvin pot 280 is thermally insulated from the helium tank 276 of the magnet. To this end, the radiation shield 270 and the outer vacuum container of the main magnet 266 have an extension into the cavity containing the 1 Kelvin pot 280. The 1 Kelvin pot 280 also needs a high-vacuum insulation sleeve 274 going all the way around, to insulate the cold helium on the inside from the warm inner wall of the cavity of the magnet. Although the embodiments shown in Figs. 2c and 2d are more complex to build, this allows the 1 Kelvin pot 180 to be warmed on its inside or even extracted from the magnet without warming the main magnet. Fig. 2c shows this scheme when mounted at the top of the main magnet. In Fig. 2d, the 1 Kelvin pot 280 is mounted below the subject support of the MRI magnet. If the sample tube runs along the vertical part of the 1 Kelvin pot 280, this route approximates the direction of the magnetic field. Note that the field of the magnet has rotational symmetry, so a zone where the field is suitable for locating the polarizer can be found at any angular position.

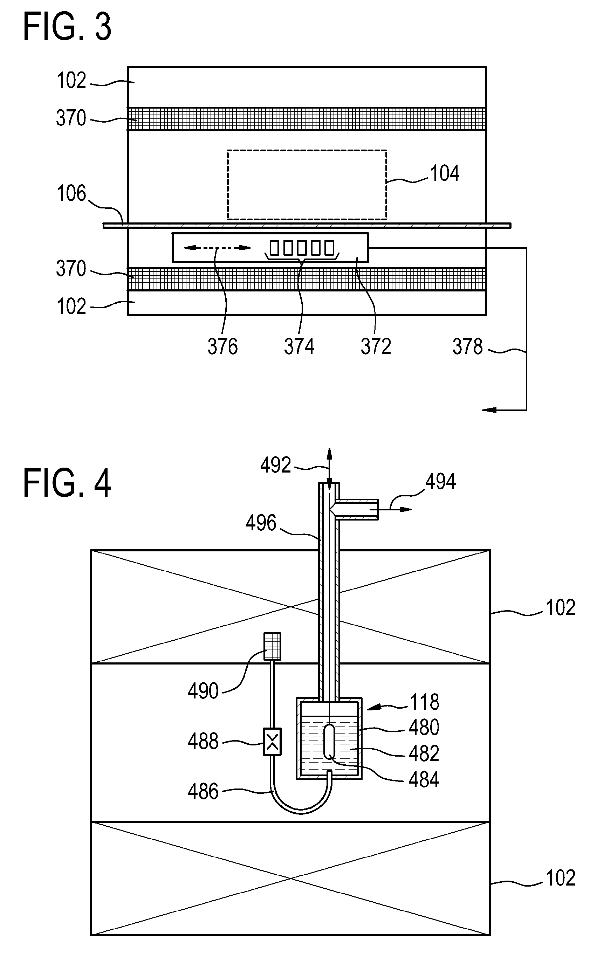

Fig. 3 shows a portion of a magnetic resonance imaging system where the polarizer 372 is located within a drawer. A cross-sectional view of a cylindrical magnet 102 is shown. Item 370 represents both the magnetic field gradient coil and the coil for the radio frequency system. Inside the drawer 372, which contains the polarizer, are samples 374 containing hyperpolarizable contrast agent. These are individual samples or cartridges which are able to be moved into the polarizer. Arrow 376 shows the direction of motion of the samples 374 within the drawer 372 of the polarizer. The subject 104 lies on a subject support 106. In this case the drawer 372 which contains the polarizer is below the subject support 106. Additionally there is a connection 378 going from the drawer of the polarizer 372 to a nuclear magnetic resonance spectrometer, a compressor, and a microwave source. The imaging zone 108 and the polarization field region 118 are not shown in these drawings. However it is understood that the subject 104 is located within the imaging zone and that in the area immediately under the subject 104, there is also a high and uniform magnetic field. In this case the imaging zone and the polarization field region may overlap.

The polarizer and sample space are designed as a drawer 372. This drawer 372 could be for example located under the patient bridge inside the homogeneous B₀ region or imaging region. In this case the polarization field region is a portion of the imaging region. The cooling facilities of the magnet (liquid Helium bath etc.) are partly used, but also a separate cooling system for the polarizer may be integrated to obtain the required lower sample temperature. During the polarization process, lower temperatures than the evaporation temperature of He are essential to boost the polarization rate. A couple of solutions are available to achieve this (e.g. evaporation pressure reduction etc.). The functionality for polarization and storage of the sample may be integrated in the same cooling chamber. A multi channel MR spectrometer may be used simultaneously for acquiring MRI data and measurements of the level of the polarization of the samples. An Electron Paramagnetic Resonance (ESR) spectrometer may also be controlled via the same spectrometer and software. However, different RF components (resonators / amplifiers) may be used for the polarizer. In parallel to the monitoring process the actual polarization process is performed that can also be controlled by a software running in parallel on the MRI system. The software may show and/or control the status of the sample and the samples in the storage. Samples are individually radiated or moved continuously through a polarization production line, where microwave irradiation is performed inside the static homogeneous B₀ field.

Fig. 4 is used to illustrate the connection between the polarizer 118 and the cooling system of a magnet 102. Since this figure is to illustrate the connection of the cooling systems, those components of the polarizer 118 not related to the cooling system are not shown. Inside the bore of the magnet 102 is located a 1 Kelvin pot 480. Within the 1 Kelvin pot is liquid helium 482. Within the liquid helium is some hyperpolarizable contrast agent 484. There is an access tube 496 which can be used by a sample transfer mechanism 492 for inserting and removing the hyperpolarizable contrast agent 484. The access tube 496 is connected to a pump 494. The magnet 102 is connected to a filter 490 for filtering liquid helium. The filter is connected to a capillary 486 which runs between the filter and the 1 Kelvin pot 480. A valve 488 in the capillary 486 controls the flow of liquid helium from the magnet 102 to the 1 Kelvin pot 480.

Fig. 4 shows how a polarizer such as a DNP polarizer may be located in the bore of a MRI magnet. The capillary may be attached to the helium tank of the magnet and routed through the vacuum space in a way that does not create a thermal short in the MRI magnet and emerge in the bore of the magnet 102. The tube may require high-vacuum insulation to achieve this. Routing an exhaust tube of the 1 Kelvin pot 480 may require another thermally insulated passage through the entire cryostat of the magnet 102. Liquid Helium 482 at 4.2 K is directly transferred from the magnets helium reservoir via a capillary 486 to the polarization chamber. A remotely located vacuum pump reduces the vapor pressure so that the Helium temperature inside the DNP chamber (1 Kelvin pot 482) may be lowered to a temperature lower than the liquid helium inside of the MRI magnet. Thermal radiation shields and a separate vacuum chamber surrounding the DNP chamber (not shown in the picture) are necessary for proper operation.

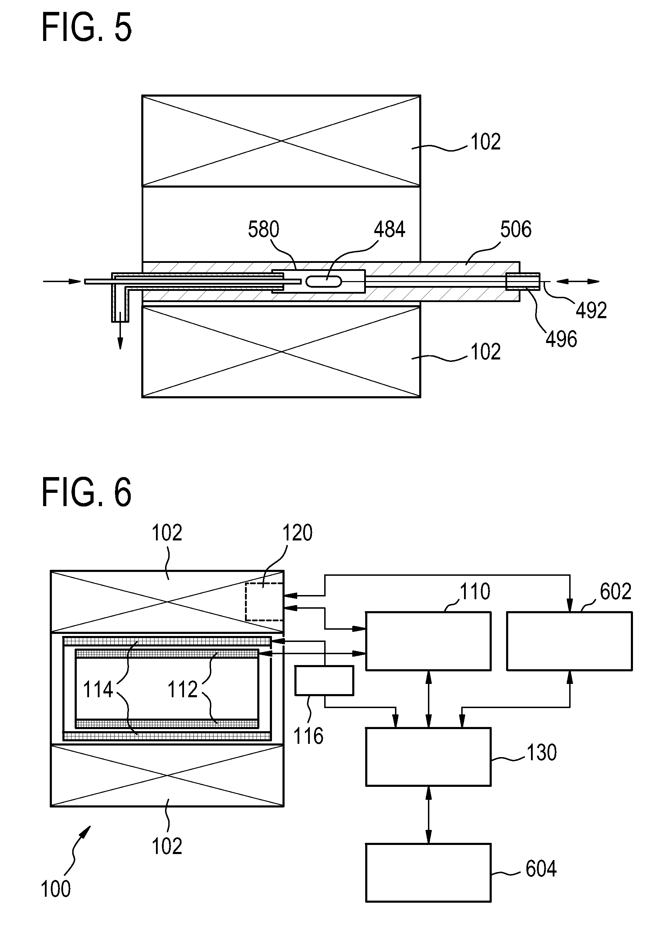

Fig. 5 shows an example of how a polarizer can be integrated into a subject support 506. Shown in Fig. 5 is a magnet 102 of a magnetic resonance imaging system. Within the magnet is a subject support 506. There is a 1 Kelvin pot 580 located in the center of the subject support 506. Within the 1 Kelvin pot is located a sample of hyperpolarizable contrast agent 484. Again there is an access tube 496 for inserting and removing the hyperpolarizable contrast agent 484. In this figure, the access tube 496 is shown as being horizontal all the way to the room temperature end. However, in such a situation liquid helium may be able to flow out of the access tube 496. In an actual implementation a section of the access tube may have a bend or be vertical to prevent liquid helium from flowing out. A sample transfer mechanism 492 is used to move the hyperpolarizable contrast agent through the access tube 496. There is a connection between the external liquid helium reservoir 500 and the 1 Kelvin pot 580. Additionally there is a connection between the pump 494 and the 1 Kelvin pot 580. Again in Fig. 5 the connections to the cooling system are emphasized and the individual parts of the polarizer 118 are not shown.

Fig. 5 illustrates how a polarizer such as a DNP polarizer may be integrated in a subject support. The subject support may also be called a patient bed. The polarizer is connected to a separate external He reservoir 500 and an external vacuum pump. Additional vacuum chambers and thermal radiation shields (not shown in the picture) may be necessary for stable operation.

Fig. 6 shows a functional diagram of a magnetic resonance imaging system 100 according to an embodiment of the invention. Shown is a magnet 102. There is a polarizer 120 integrated into the magnet 102. Within the bore of the magnet are shown magnetic field gradient coils 114 and coils 112 for the radio frequency system. The coils of the radio frequency system 112 are connected to the radio frequency system 110. The magnetic field gradient coils 114 are connected to the magnetic field gradient power supply 116. Additionally the polarizer 120 is connected to the radio frequency system 110 and to an electron paramagnetic resonance spectrometer 602. The radio frequency system uses a nuclear magnetic resonance coil in the polarizer 120 for measuring the degree of polarization. The electron paramagnetic resonance spectrometer 602 is also used to monitor the degree of polarization of the single electron substance or the hyperpolarizable contrast agent within the polarizer 120. The magnetic field gradient power supply is controlled by a computer system 130. The radio frequency system 110 is also controlled by the computer 130 and the electron paramagnetic resonance spectrometer 602 is also controlled by the computer 130. There is also a display 604 attached to the computer 130 for the visualization of magnetic resonance imaging images.

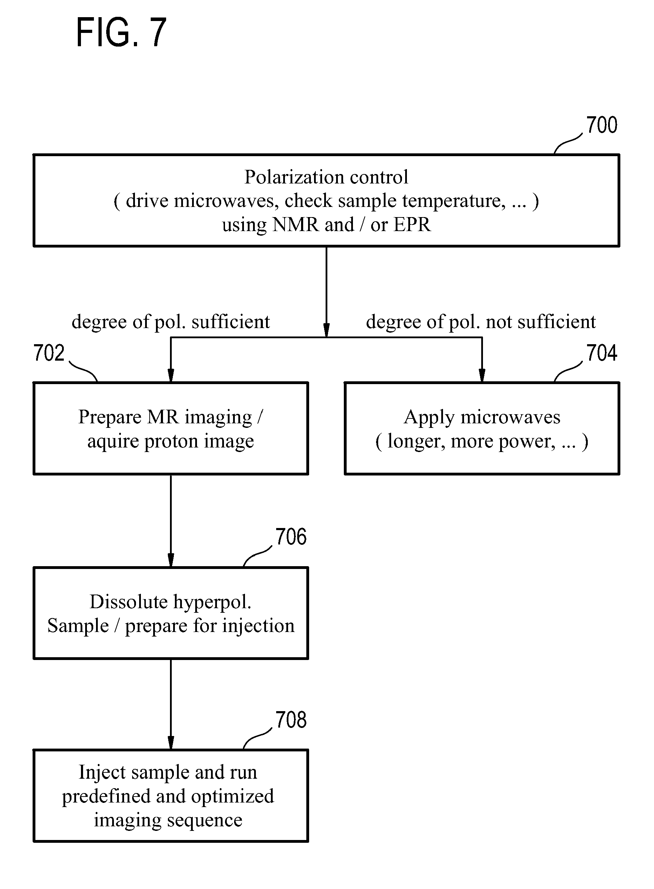

Fig. 7 shows a method of operating a magnetic resonance imaging system according to an embodiment of the invention. This method may be performed manually or may be performed in an automated manner by a computer using a computer program product. In step 700 polarization control is used to polarize the hyperpolarizable contrast agent. Nuclear magnetic resonance or electron paramagnetic resonance is used to monitor the degree of polarization. The method then splits into two branches. If the degree of polarization is not sufficient in step 704 microwaves are applied for longer or more power is used. If the degree of polarization is sufficient then step 702 is performed. In this step the magnetic resonance imaging of the hyperpolarizable contrast agent is prepared by first acquiring a proton image of the subject in the imaging zone. In step 706 the hyperpolarizable contrast agent is dissolved and prepared for injection or administration to the subject. In step 708 samples of the hyperpolarizable contrast agent are administered to the subject and an optimized imaging sequence for acquiring magnetic resonance imaging data of hyperpolarizable contrast agent in the subject is performed.

Fig. 7 sketches a possible workflow for imaging using an embodiment of the invention. The sample state is permanently controlled and microwave irradiation as well as temperature is monitored and adjusted 700. Once the degree of polarization of the sample is sufficient, imaging is prepared 702 (e.g. by carrying out a reference scan, by acquiring a proton image or any other preparation measure). The sample is then manually or automatically dissolved and prepared for injection 706. The magnetic resonance imaging system or MR scanner may then trigger the injection and start an optimized imaging or spectroscopy sequence on the ¹³C (or other) frequency 708.

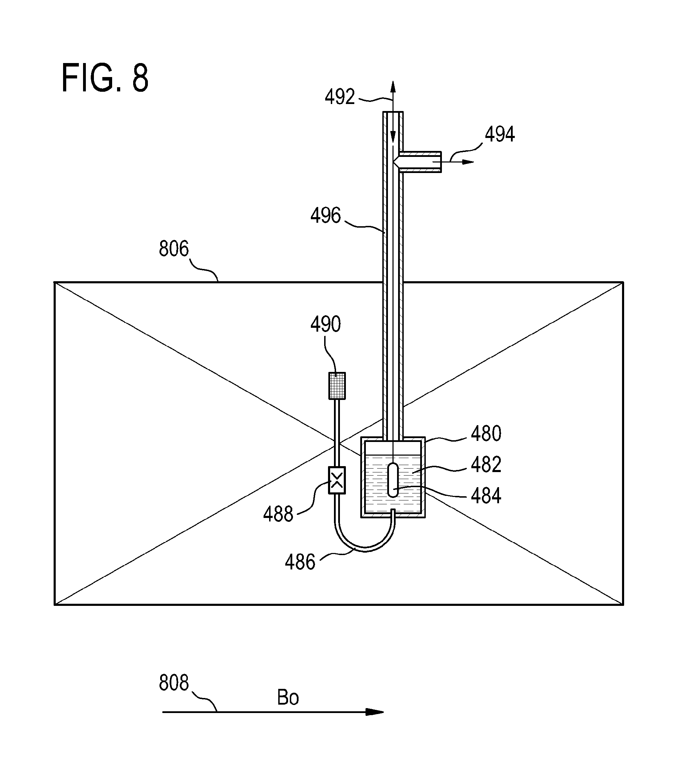

Fig. 8 illustrates the connection between the polarizer 118 and the cooling system of a magnet 102 in the same way as was illustrated in Fig. 4. However in Fig. 8 the polarizer is located within a portion of the magnet 806. In this case only a portion of a cross sectional view of a cylindrical magnet is shown. Arrow 808 indicates the orientation of the magnetic field of the magnet 806.

Fig. 8 shows how a polarizer such as a DNP polarizer may be located directly inside the MRI magnet 806. Liquid Helium at 4.2 K is directly transferred from the magnets helium reservoir via a capillary 486 to the polarization chamber. A remotely located vacuum pump 494 reduces the vapour pressure so that the Helium 482 temperature inside the DNP chamber (1 Kelvin pot 480) may be lowered to a temperature lower than the liquid helium in the MRI magnet.Thermal radiation shields or extra vacuum chambers are not necessary.

Another embodiments of a combined MRI magnet and polarizer is where the helium reservoir and helium supply of the polarizer is not directly drawn from the reservoir of the main magnet. In this case, the polarizer may have an external supply of helium. The external supply of helium could for example be a storage tank located close to but not integrated with the cryostat of the magnet. Over time, helium from the storage tank will be evaporated in the polarizer. This evaporation may be due to thermal heat leakage, as a result of pumping down to 1K, and due to dissipation by the polarization and sample extraction processes. The gas escaping from the polarizer may be slightly pressurized and may be routed to the top of the helium tank of the main magnet. Assuming the magnet is equipped with a 4K helium refrigerator with some excess cooling capacity (as is nowadays state of the art), at least a fraction of this gas could be recondensed by the magnet's refrigerator and will be accumulated in the helium tank of the magnet. This additional liquid could be kept there as a buffer (to be used for example to compensate for the losses of ramping the magnet) or it could be harvested at regular intervals by syphoning it back into a storage dewar (possibly the buffer tank of the polarizer).

### LIST OF REFERENCE NUMERALS:

- 100: Magnetic resoance imaging system
- 102: Magnet
- 104: Subject
- 106: Subject support
- 108: Imaging region
- 110: Radio frequency system
- 112: Coil
- 114: Magnetic field gradient coil
- 116: Magnetic field gradient power supply
- 118: Polarization field region
- 120: Polarizer
- 122: Ferromagnetic structure
- 124: Tube between polarizer and hyperpolaizable contrast agent dispenser
- 126: Hyperpolarizable contrast agent dispenser
- 128: Intravenous connection between subject and hyperpolarizable contrast agent dispenser
- 130: Computer system
- 132: Hardware interface
- 134: User interface
- 136: Microprocessor
- 138: Storage
- 140: Memory
- 142: Computer program product
- 144: Image construction module
- 146: Magnetic resoance imaging system control module
- 148: Hyperpolarizable contrast agent dispenser control module
- 150: Polarizer control module

- 152: Computer program product
- 154: Archive of patient data
- 156: Inner wall
- 158: Outer wall
- 160: Cavity
- 260: Superconducing coil
- 262: Magnetic field lines
- 264: Superconducing coils with field lines drawn
- 266: Wall of magnet
- 268: Exterior wall
- 270: Radiation shield
- 272: Interior wall
- 274: Vaccum
- 276: Interior of magnet
- 278: Entrance to 1 Kelvin pot
- 279: Tube
- 280: 1 Kelvin pot
- 282: Path for extracting hyperpolarizable contrast agent
- 284: Gas outlet
- 370: magnetic field gradient coil and coil
- 372: Polarizer in a drawer
- 374: Hyperpolarizable contrast agent samples
- 376: Arrow showing direction of motion of hyperpolarizable samples in drawer
- 378: Connection to nuclear magnetic resoance spectrometer, compressor, and microwave source
- 480: 1 Kelvin Pot
- 482: 1.2K liquid He
- 484: Hyperpolarizable contrast agent
- 486: Capilary

- 488: Valve
- 490: Filter
- 492: Sample transfer mechanism
- 494: Connection to pump
- 496: Access tube
- 500: Connection to external liquid He reservoir
- 506: Patient support
- 580: 1 Kelvin Pot
- 602: Electron paramagnetic resonance spectrometer
- 604: Display for visualization
- 806: Portion of cylindrical magnet
- 808: Direction of magnetic field B₀

## Claims

1. A magnetic resonance imaging system (100) adapted for acquiring magnetic resonance imaging images comprising:
- a magnet (102) adapted for generating a magnetic field (262) for orientating the magnetic spins of nuclei of a subject (104) located within an imaging region (108),
- a polarizer (120) adapted for polarizing a hyperpolarizable contrast agent (484), wherein the polarizer is located within a polarization field region (118) of the magnetic field of the magnet,
- a radio frequency system (110) comprising a coil (114) adapted for acquiring magnetic resonance imaging data from the hyperpolarizable contrast agent,
- a magnetic field gradient coil (114) adapted for spatial encoding of the magnetic spins of nuclei,
- a magnetic field gradient coil power supply (116) adapted for supplying current to the magnetic field gradient coil,
- a computer system (130) adapted for constructing images from the magnetic resonance imaging data and for controlling the operation of the magnetic resonance imaging system.

2. The magnetic resonance imaging system of Claim 1, wherein the polarizer comprises a nuclear magnetic resonance coil adapted for receiving nuclear magnetic resonance data from the hyperpolarizable contrast agent, and wherein the radio frequency system is adapted for acquiring nuclear magnetic resonance data from the hyperpolarizable contrast agent using the nuclear magnetic resonance coil.

3. The magnetic resonance imaging system of Claim 1or 2, wherein the magnetic resonance imaging system further comprises an electron paramagnetic resonance spectrometer (602) for monitoring the polarization of the hyperpolarizable contrast agent.

4. The magnetic resonance imaging system of Claim 1, 2 or 3, wherein the polarizer is further adapted for being connected to a hyperpolarizable contrast agent dispenser (126).

5. The magnetic resonance imaging system of Claim 4, wherein the computer system is further adapted for sending control signals to the dispenser, wherein the computer system is adapted for controlling the polarization of the hyperpolarizable contrast agent, wherein the computer system is further adapted for controlling the dispensing of hyperpolarizable contrast agent to the subject using the polarizer and the hyperpolarized contrast agent dispenser.

6. The magnetic resonance imaging system of any one of the preceding Claims, wherein the computer system is further adapted for controlling the polarizer, wherein the computer system comprises a display, wherein the computer system is further adapted for displaying the polarization level of the hyperpolarizable contrast agent in the polarizer.

7. A magnet adapted for generating a magnetic field (262) for orientating the magnetic spins of nuclei of a subject (104) located within an imaging region (108) of a magnetic resonance imaging system, wherein the magnet further comprises a polarizer (120, 372) adapted for polarizing a hyperpolarizable contrast agent (374), wherein the polarizer is located within a polarization field region (118) of the magnetic field of the magnet.

8. The magnet of Claim 7, wherein the magnet comprises a patient support, wherein the polarizer is located below the patient support.

9. The magnet of Claim 7 or 8, wherein the magnet comprises a drawer (372) adapted for holding the polarizer and/or adapted for storing hyperpolarizable contrast agent.

10. The magnet of Claim 7, 8, or 9, wherein the polarizer comprises a 1 Kelvin pot (480, 580), and wherein the magnet comprises a cryogenic cooling system for cooling the magnet and the 1 Kelvin pot.

11. The magnet of any one of Claims 7 though 10, wherein the magnet further comprises a ferromagnetic structure (122) that follows a path at least partially between the imaging region and the polarization field region, and wherein the ferromagnetic structure is adapted for minimizing the magnetic field B₀ gradient between the polarization field region and the imaging region and/or the ferromagnetic structure is adapted for enhancing a local region of the magnetic field between the polarization field region and the imaging region.

12. The magnet of any one of Claims 7 through 11, wherein the magnet comprises a cryostat, wherein the polarization field region is within the cryostat.

13. The magnet of any one of Claims 7 through 12, wherein the magnet is further adapted such that the magnitude of the magnetic field generated in the polarization field region is stronger than magnitude of the magnetic field generated in the imaging region.

14. A subject support (506) for a magnetic resonance imaging system, wherein the subject support comprises a polarizer (580) adapted for polarizing a hyperpolarizable contrast agent (484).
